# EUROPEAN PATENT SPECIFICATION

(11) **EP 2 488 653 B1**
(45) Date of publication and mention of the grant of the patent: **28.08.2013**
(21) Application number: 09748848.0
(22) Date of filing: 16.10.2009
(51) Int. Cl.: C12P 7/62

(54) **Using cell debris generated from PHA recovery for enhanced cell growth**
Verwendung von Zelltrümmern aus der PHA-Wiedergewinnung für verbessertes Zellwachstum
Utilisation de débris cellulaires générés par la récupération de pha pour la croissance améliorée de cellules

(43) Date of publication of application: 22.08.2012
(73) Proprietor: Bio On S.r.l., 40016 San Giorgio Di Piano (Bologna) (IT)
(72) Inventor: YU, Jian, Honolulu, Hawaii 96825 (US)
(74) Representative: Bottero, Carlo
(86) International application number: PCT/IB2009/007142
(87) International publication number: WO 2011/045625

(56) References cited:
- RAHAYU ET AL: "Production of copolymer poly(3-hydroxybutyrate-co-4-hydroxybutyrat e) through a one-step cultivation process" WORLD JOURNAL OF MICROBIOLOGY AND BIOTECHNOLOGY, vol. 24, 6 May 2008 (2008-05-06), pages 2403-2409, XP019650334
- ZHANG ET AL: "Microbial production of 4-hydroxybutyrate, poly-4-hydroxybutyrate, and poly(3-hydroxybutyrate-co-4-hydroxybutyrat e) by recombinant microorganisms" APPLIED MICROBIOLOGY AND BIOTECHNOLOGY, vol. 84, 12 May 2009 (2009-05-12), pages 909-916, XP019737741

## Description

### Background of the invention

The present invention relates to a process for producing biodegradable polymeric materials from an organic carbon source, e.g. sugar beet pulp and molasses. Particularly, the present invention relates to a process for producing biodegradable polymeric materials including polyhydroxyalkanoates with cell debris, a type of organic waste left from recovery and purification of polyhydroxyalkanoates from cells.

Polyhydroxyalkanoates (PHAs) are homopolymers or copolymers of hydroxyalkanoates, such as 3-hydroxybutyrate (3HB), 3-hydroxyvalerate (3HV), 4-hydroxyvalerate (4HV) and 3-hydroxyhexanoate (3HH). These thermoplastic or elastic biopolymers are synthesized and accumulated by many microorganisms, bacteria in particular, as carbon and energy storage materials. PHAs are conveniently synthesized by cultivating the microbial cells in an aqueous medium on a carbon source, including sugars, organic acids and alcohols. Depending on strains and growth conditions, the biopolyesters in a form of small granules (0.3-0.5 µm) may account for 50-80 wt% of dry cell mass.

The PHA granules should be separated from the rest non-PHA cell mass so that the bioplastics have the desired purity and material property. The residual cell debris from PHA recovery, consisting of proteins, nucleic acids, lipids and wall fragments, has no market value and is either discarded as a solid waste or discharged in process wastewater.

Two technologies are usually used for PHA recovery, based either on organic solvent extraction of polyesters or digestion of non-PHA cell mass. In the former, PHA granules are dissolved in appropriate organic solvents, leaving cell residue solids. In the latter, PHA granules are left as solids, but non-PHA cell mass is digested and dissolved in aqueous solutions with help of biological and chemical agents. A conventional solid/liquid separation following the treatments generates a stream of PHA product and another stream of non-PHA cell debris. According to cell composition , the cell debris is actually the true cell mass containing all the essential components from cell growth, while product PHA is only a carbon reserve material. In order to produce PHA polyesters, sufficient nutrients (C, N, P, minerals and organic growth factors) must be consumed to grow enough cells that in turn synthesize biopolyesters from the carbon source.

U.S. Patent No. 7,514,525 relates to a method to recover, purify and isolate PHA biopolymers from PHA-containing cell mass, which includes: (a) solubilizing the non-pHA cell mass in an acidic solution, leaving a suspension of partially crystallized PHA granules; (b) adjusting the pH of the suspension to 7-11 and separating the PHA solids from the dissolved non-PHA cellular mass; (c) resuspending the PHA solids in a bleaching solution for decolorization; and (d) drying the resulting PHA solids. About 95% or greater of original PHA in cell mass is recovered, and the purity of PHA solids is about 97% or above.

### Summary of the invention.

The Applicant has faced a problem of improving the yield of a process for producing PHAs from an organic carbon source by microbial fermentation. The Applicant has also faced the problem of disposing the cell debris left from PHA recovery and purification. The Applicant has found that the above problems can be solved by using the cell debris obtained upon solubilizing the non-PHA cell mass to further feed the PHA producing microorganisms. Reuse of the above cell debris as nutrients remarkably increases the yields of cell growth and PHA synthesis on carbonaceous substrates such as glucose, fructose and sucrose, since it can be readily assimilated by the microbial cells.

Therefore, according to a first aspect, the present invention relates to a process for producing biodegradable polymeric materials including polyhydroxyalkanoates (PHAs) from an organic carbon source, which comprises:
(a) cultivating PHA-producing microbial cells in a medium solution containing an organic carbon source to form PHAs that are accumulated in the cells as inclusion bodies;
(b) harvesting the cells from the spent medium and solubilizing the non-PHA cell mass to obtain a PHA solid and a cell debris solution;
(c) separating the PHA solid from the cell debris solution;
(d) feeding the cell debris solution to the cultivation step (a).

### Detailed description of the invention.

With "organic carbon source" it is meant any organic compound or mixtures thereof which can be metabolized by PHA-producing microbial cells, such as glucose, fructose, sucrose or similar carbohydrates or any organic mixtures of carbohydrates such as sugar beet pulp, sugar beet molasses, sugar cane molasses. The medium solution, besides the organic carbon source, may contain additional organic growth factors, N, P and/or other minerals as nutrients for the cell growth.

According to a preferred embodiment, the solubilizing step (b) is carried out by:
(b1) solubilizing the non-PHA cell mass in an acidic solution to obtain a first suspension of a PHA solid in an acidic cell debris solution;
(b2) adjusting the pH of the first suspension to a value of 7 to 11 to obtain a second suspension of the PHA solid in a basic cell debris solution.

According to the above preferred embodiment, the cells containing PHA granules are first treated in an acidic solution releasing a substantial portion of proteins (acidic cell debris). At the same time, the PHA granules are partially crystallized from the original labile structure, which makes the polyesters tough and resistant to chemical digestion. The acidic treatment may be carried out by adding an aqueous solution of a strong acid, such as sulfuric acid. Preferably, the aqueous solution of a strong acid is added to the non-PHA cell mass in an amount so as to achieve a concentration of hydrogen ions (H⁺) from 0.01 to 0.5 mole/L. The solubilizing step (b1) is preferably carried out at a temperature from 80° to 130°C, for a time from 0.5 to 5 hours.

After separation from the acidic solution, the cells with PHA granules are further treated in a base solution to dissolve the rest non-PHA cell mass. Preferably, the base solution is an aqueous solution of at least one strong base, such as sodium hydroxide or potassium hydroxide.

The alkaline treatment may be assisted by adding at least one surfactant, preferably at least one ionic surfactant, preferably a C₆-C₁₈ alkyl sulfate, such as sodium dodecyl sulfate (SDS). The at least one surfactant promotes cell disruption and membrane decomposition. The at least one surfactant is preferably added in an amount of from 2 to 10 g/L, preferably from 4 to 7 g/L.

The above two-step treatment digests and dissolves most of non-PHA cell mass while little PHA is lost. Equally important, the treatments can convert the cell debris into appropriate forms that can be directly assimilated by microbial cells as nutrients for growth and PHA formation.

Three types of cell debris solutions may be generated in PHA recovery as aqueous wastes:
1. Acidic cell debris solution (ACDS) that contains proteins and other soluble biological compounds released from cells in acidic solution.
2. Basic cell debris solution (BCDS), optionally including at least one surfactant, that contains amino acids, fatty acids and other digested cell components derived from the acid-treated cell residues under alkaline conditions.
3. Acid-base cell debris solution (ABCDS), optionally including at least one surfactant, that contains amino acids, fatty acids, peptides and other digested cell components in both acid and base conditions. This aqueous waste solution is generated when the sequential acid and base treatments are performed without cell separation.

According to the present invention, these three types of cell debris may be reused as nutrients in microbial production of PHA biopolyester.

Therefore, according to a first preferred embodiment, in the process of the present invention a separating step (c1) is carried out on the first suspension and the resulting acidic cell debris solution is fed according to step (d).

According to a second preferred embodiment, in the process of the present invention a separating step (c2) is carried out on the second suspension and the resulting basic cell debris solution is fed according to step (d).

Preferably, both the separating step (c1) and the separating step (c2) are carried out on the first suspension and on the second suspension respectively, and the resulting cell debris solutions are fed according to step (d).

According to a third preferred embodiment, in the process of the present invention a separating step (c3) is carried out only on the second suspension and the resulting acid-base cell debris solution is fed according to step (d).

Preferably, the total amount of cell debris that is co-fed with the organic carbon source to the cultivation step (a) is from 5 to 50% by weight, preferably from 10 to 40% by weight, with respect to the weight of the glucose-equivalent carbon substrate. With "weight of the glucose-equivalent carbon substrate" it is meant the amount of the carbon organic source expressed by the corresponding amount of glucose as substrate for the microbial fermentation.

In the case of the acidic cell debris solution (ACDS) fed to step (a), the amount of cell debris is preferably from 5 to 20% by weight with respect to the weight of the glucose-equivalent carbon substrate. In fact, an inhibition effect by the ACDS has been observed on cell growth and PHA formation when the above amount is greater than 20% by weight. Such an effect has not been observed when feeding the BCDS or the ABCDS.

The PHA solid obtained from the separation step (c) may be subjected to further treatments, such as decolorization, washing and/or drying for achieving the desired product purity and quality according to known techniques, such as those reported in the above-cited U.S. Patent 7,514,525.

The following working examples are given to better illustrate the invention, but without limiting its scope.

### Cell debris solutions.

After PHA fermentation, the cells containing PHA granules were harvested from medium with centrifugation at 5,000 g for 10 min. The wet cell pellets had a density of 450-500 g dry mass/L depending on medium volume, centrifugation force and time. PHA were recovered and purified from the cells by removing the non-PHA cell mass (about 30% w/w) in three steps: (1) acid pretreatment, (2) base treatment, (3) hypochlorite decolorization, and (4) washing and drying. They are detailed as follows.
(1) Acid pretreatment: The wet cell pellets from fermentation broth were resuspended in an equivalent volume of 0.2M H₂SO₄ aqueous solution. The slurry of 200-250 g dry matter/L was heated to boil and then maintained for one hour under ambient conditions. After cooling to room temperature, the acid pretreated pellets were separated from the acidic solution with centrifugation at 5,000 g for 10 min. The clean supernatant had a brownish color and a solid content between 35-65 g/L depending on the density of slurry and treatment conditions. It is referred as acidic cell debris solution (ACDS) thereafter.
(2) Base treatment: The wet cell pellets from the acid pretreatment were re-suspended in an equivalent volume of water that was pre-adjusted to pH 10.2 to 10.5 with 5 M NaOH solution. A small amount of surfactants such as sodium dodecylsulfate (SDS, CH₃(CH₂)₁₁OSO₃Na) was added to a concentration of 5 g/L and the slurry was stirred in ambient conditions for 30 min. It was heated to boiling and kept under ambient conditions for 10 min. The treated PHA/cells were separated from a dark supernatant after centrifugation at 5,000 g for 15 min. The supernatant solution had a solid content of 30 to 60 g dry matter/L and is referred as basic cell debris solution (BCDS) thereafter.
(3) The sequential acid and base treatments above could also be performed without cell separation after the acid treatment. A base such as sodium hydroxide and a surfactant such as SDS were directly added into the acidic cell slurry and the pH was raised to 10-10.5 for base treatment. An acid-base cell debris solution, referred as ABCDS thereafter, was generated with solid/liquid separation following the base treatment. The aqueous wastes contained two types of cell debris dissolved in sequential acid and base treatments.
(4) Hypochlorite decolorization: The wet pellets from base treatment were re-suspended in a commercially available bleaching solution containing 6% w/w of hypochlorite. The volume of bleaching solution was estimated based 1 part of hypochlorite for 1 part of PHA-containing dry solid. The amount of PHA solid was estimated from the wet mass and its dry solid content of 60% w/w. The slurry was stirred for 2 hours under ambient conditions. A white PHA pellets were recovered after centrifugation at 5,000 g for 20-30 min. The supernatant solution contained residual hypochtonte was reused for bleaching after fresh hypochlorite was added.
(5) Washing and drying: The wet white PHA solids from bleaching were washed with water for two times and dried in oven at 80°C. The final PHA product was a white powder.

The following scheme shows an example of PHA recovery and purification and discharge of acidic and basic cell debris solutions. It started with 0.3 L cell slurry containing 83.4 g dry cell mass (DM) with a PHA content of 72% w/w. After treatment with removal cell debris, the final PHA powder contained 96.4% of PHA.

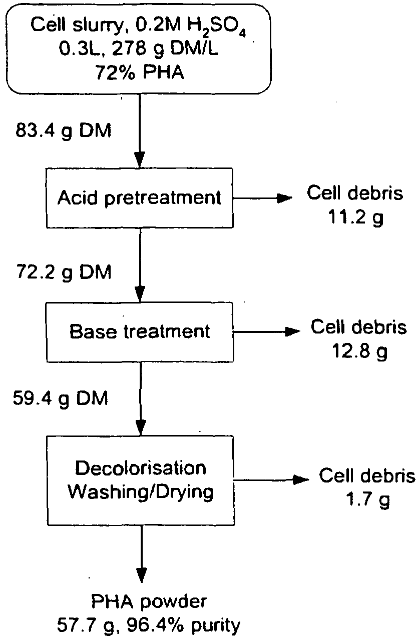

After acid pretreatment, 72.2 g dry solid was recovered and 11.2 g cell debris was dissolved in acid solution and discharged. With base and SDS treatment, 59.4 g dry solid was recovered and 12.8 g cell debris was dissolved in base solution and discharged. A small amount of PHA polymers were lost in cell debris because of separation loss. The cell debris discharged from acid and base treatments accounted for more than 90 wt% of the total cell debris discharged from PHA recovery and purification process.

### Using cell debris in PHA fermentation.

In PHA recovery as shown above, the non-PHA cell mass including proteins, nucleic acids, membrane lipids and cell wall fragments were decomposed to cell debris that was dissolved as soluble solids in aqueous solutions.

Starting with cell mass of high PHA content (72% w/w), production of 1 kg of PHA, in a purity of 96.4 w/w, generates about 0.45 kg of cell debris as shown in the scheme reported above. Disposal of this large amount of waste in aqueous solution is a task of high cost, but could be avoided with additional benefit if the cell debris could be reused in PHA fermentation. In order to reveal the effect of cell debris on cell growth and PHA formation, microbial cultures were conducted in 500 mL flasks in parallel. The flask cultures were set up with the same glucose concentrations (10-20 g/L), the same volume of mineral solution (160 mL) as shown in Table I reported hereinbelow, and the same inoculum and size. After predetermined amount of cell debris solutions were added, the total volume of flask cultures was raised to 200 mL by adding presterilized distilled and deionized water. The flasks were shaken at 200 rpm and 30 °C in a rotary incubator for 24 or 48 hours. The cell mass concentration and PHA content were determined as described in the literature (see Jian Yu and Lilian X. L. Chen (2006), "Cost-effective recovery and purification of polyhydroxyalkanoates by selective dissolution of cell mass", Biotechnology Progress, 22:547-553).

**TABLE 1 - Mineral solutions for cell growth and PHA synthesis**

| Mineral medium (a) | Concentration (g/L) | Trace solution (b) | Concentration (g/L) |
|---|---|---|---|
| NaH₂PO₄ | 2 | Fe(NH₄)citrate | 50 |
| K₂BPO₄ | 2.8 | CaCl₂.H₂O | 7.8 |
| MgSO₄.7H₂O | 0.5 | ZnSO₄.7H₂O | 0.5 |
| (NH₄)₂SO₄ | 1 | MnSO₄.H₂O | 0.1 |
| Trace solution (b) | 1 mL | CuCl₂.2H₂O | 0.05 |
| | | Na₂MoO₄.2H₂O | 0.15 |
| | | H₃BO₃ | 1.5 |
| | | CoSO₄.7H₂O | 1.1 |
| | | NiCl₂.6H₂O | 0.1 |

| | | | |
|---|---|---|---|
| (a) The mineral solution is autoclaved at 121°C for 10 min. (b) The trace solution is prepared in 0.1 N HCI and directly added, without sterilization, into the autoclaved mineral medium. | | | |

### Case 1. Using acidic cell debris solution (ACDS)

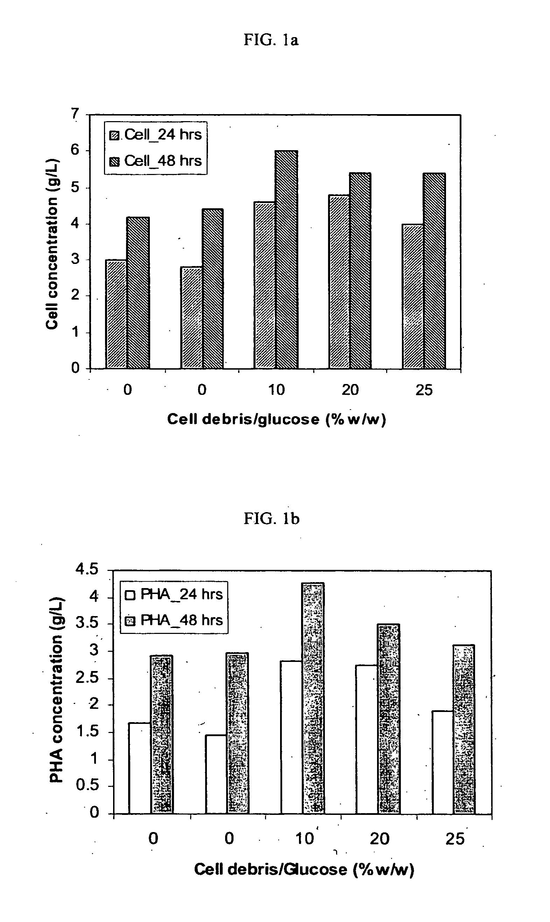

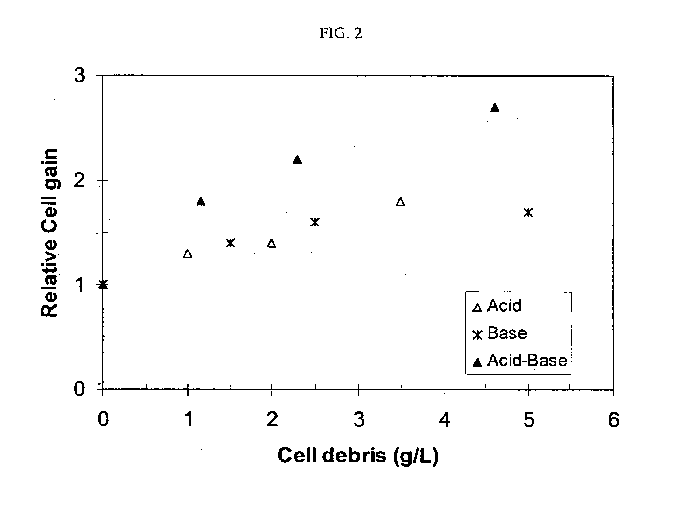

An acidic cell debris solution containing 38 g/L of soluble solids was used in this experiment. Predetermined amount of ACDS was added into the flasks to give different ratio of cell debris to glucose at 0, 10, 20 and 25% of sugar, respectively. Duplicates without cell debris were run in parallel as experimental controls. As shown in Figure 2, the cell debris is clearly beneficial to cell growth and PHA synthesis. The benefits are statistically significant in comparison with two controls. First, the cell debris leads to a faster cell growth rate than the controls in the first 24 hours. Secondly, the cell debris can also be used as an extra carbon source generating more cells than controls in 48 hours. Thirdly, the cell debris has the same positive effect on PHA synthesis. Fourthly, there is an optimal ratio of cell debris to glucose (10% w/w in this case). Too much cell debris could inhibit cell growth and PHA formation as shown in Figure 2.

In this demonstration, the overall cell growth yield (Yx/s) and PHA formation yield (Yp/s) are calculated from the amounts of cell mass and PHA formed and the amount of sugar initially added, regardless the amount of sugar unused. The benefits of reusing cell debris in PHA fermentation are clear and substantial when the yields are compared with those of controls as shown in Table 2. The differences of the relative yields are statistically meaningful. Generally speaking, at the dosage of cell debris of around 10% w/w of sugar, the cell growth has a gain of 40-50% and PHA formation has a gain of 45-65% w/w in-comparison with the controls. The yields are relative to the controls without cell debris addition.

**TABLE 2. Effects of acidic cell debris on the yields of cell mass and PHA formation.**

| Cell debris/sugar (% w/w) | Yx/s (g cell/g sugar) | | Yp/s (g PHA/g sugar) | |
|---|---|---|---|---|
| | 24hrs | 48hrs | 24hrs | 48hrs |
| 0 | 1.00 ± 0.06 | 1.00 ± 0.05 | 1.00 ± 0.1 | 1.00 ± 0.02 |
| 10 | 1.54 | 1.43 | 1.67 | 1.46 |
| 20 | 1.60 | 1.29 | 1.63 | 1.20 |
| 25 | 1.34 | 1.29 | 1.14 | 1.07 |

### Case 2. Using acid-base cell debris solution (ABCDS)

In the same flask cultures above, an acid-base cell debris solution (48.5 g/L of soluble solids) was added to give different ratios of cell debris to glucose (0 - 40 wt%) as shown in Table 3. In comparison with the controls of no cell debris added, the concentrations of both cell mass and PHA content were substantially increased. The relative yields were increased by 100 - 300%. No inhibitory effect of acid-base cell debris was observed, probably because of the digestion of the inhibitors in alkaline treatment.

**TABLE 3. Effect of acid-base cell debris on cell growth and PHA formation in 48 hours.**

| Cell Debris (g/L) | Debris/sugar (wt%) | Cell mass (g/l) | PHA (wt%) | PHA (g/L) | Yx/s | Yp/s |
|---|---|---|---|---|---|---|
| 0.0 | 0 | 2.1 | 45 | 0.95 | 1.0 | 1.0 |
| 1.15 | 9.4 | 4 | 55 | 2.2 | 1.8 | 2.25 |
| 2.30 | 18.8 | 4.5 | 60 | 2.7 | 2.1 | 2.75 |
| 4.60 | 38.8 | 5.6 | 61 | 3.4 | 2.6 | 3.50 |

### Case 3. Comparing three types of cell debris as growth nutrients

In the same flask cultures above, three types of cell debris solutions (ACDS, BCDS and ABCDS) were added to different concentrations of cell debris. The cell mass concentrations relative to the controls without cell debris are compared after 48 hours cultivation as shown in Fig. 2. The relative cell gain reported in Fig. 2 is the ratio of a cell mass concentration to the control. The nutritional effect of ACDS and BCDS on cell growth is very similar, while ABCDS has a better nutrient value than do ACDS and BCDS.

### Case 4. Effect of surfactant in cell debris

Surfactants such as SDS are optionally used in PHA recovery to disrupt cells and remove lipids and pigments from polyesters. They are soluble and left in the cell debris solutions, which may cause adverse effect on cell growth when the cell debris solution is reused in PHA fermentation. An acid-base cell debris solution containing 48.5 g/L of soluble solids and 5 g/L of SDS was used in this case. In the same flask cultures above (12 g/L of glucose), 8 mL of ABCDS was added to give 1.94 g/L of cell debris and 0.2 g/L of SDS. Additional SDS was also added to increase SDS concentration to 0.4, 0.6 and 0.8 g/L. Table 4 gives the results of cell growth and PHA formation at different surfactant levels. Compared to the control without cell debris and surfactant, all flasks with cell debris show benefits to cell growth. PHA formation is also enhanced at low to moderate surfactant concentrations (0.2 - 0.6 g/L), but damaged to a great extent at the high SDS concentration (0.8 g/L). The benefit of cell debris at low SDS concentration (0.2-0.4 g/L) leads to 200-300% of increase in PHA concentration in comparison with the control.

**TABLE 4. Effect of SDS surfactant on cell growth and PHA formation**

| Cell debris (g/L) | SDS (g/L) | 24 hours | | | 48 hours | | |
|---|---|---|---|---|---|---|---|
| | | Cell mass (g/L) | PHA (wt%) | PHA (g/L) | Cell mass (g/L) | PHA (wt%) | PHA (g/L) |
| 0 | 0 | 2.08 | 36.8 | 0.77 | 2.7 | 44.4 | 1.20 |
| 1.94 | 0.2 | 3.69 | 49.7 | 1.83 | 4.99 | 60.6 | 3.02 |
| 1.94 | 0.4 | 3.33 | 43.7 | 1.45 | 4.62 | 57.0 | 2.63 |
| 1.94 | 0.4 | 3.22 | 39.6 | 1.27 | 4.46 | 53.8 | 2.40 |
| 1.94 | 0.6 | 2.70 | 33.9 | 0.92 | 3.51 | 50.3 | 1.77 |
| 1.94 | 0.8 | 2.59 | 17.8 | 0.46 | 3.54 | 25.8 | 0.91 |

## Claims

1. A process for producing biodegradable polymeric materials including polyhydroxyalkanoates (PHAs) from an organic carbon source, which comprises:
(a) cultivating PHA-producing microbial cells in a medium solution containing an organic carbon source to form PHAs that are accumulated in the cells as inclusion bodies;
(b) harvesting the cells from the spent medium and solubilizing the non-PHA cell mass to obtain a PHA solid and a cell debris solution;
(c) separating the PHA solid from the cell debris solution;
(d) feeding the cell debris solution to the cultivation step (a).

2. The process according to claim 1, wherein the solubilizing step (b) is carried out by:
(b1) solubilizing the non-PHA cell mass in an acidic solution to obtain a first suspension of a PHA solid in an acidic cell debris solution;
(b2) adjusting the pH of the first suspension to a value of from 7 to 11 to obtain a second suspension of the PHA solid in a basic cell debris solution.

3. The process according to claim 2, wherein a separating step (c1) is carried out on the first suspension and the resulting acidic cell debris solution is fed according to step (d).

4. The process according to claim 3, wherein a separating step (c2) is carried out on the second suspension and the resulting basic cell debris solution is fed according to step (d).

5. The process according to claim 2, wherein a separating step (c3) is carried out on the second suspension and the resulting acid-base cell debris solution is fed according to step (d).

6. The process according to anyone of the previous claims, wherein the total amount of cell debris fed to the cultivation step (a) is from 5 to 50% by weight with respect to the amount of the glucose-equivalent carbon substrate.

7. The process according to claim 3, wherein the amount of the acidic cell debris solution (ACDS) fed to step (b) is from 5 to 20% by weight with respect to the weight of the glucose-equivalent carbon substrate.

8. The process according to claim 2, wherein the adjusting step (b2) comprises adding at least one surfactant to the first suspension.

9. The process according to claim 8, wherein the at least one surfactant is a ionic surfactant.

10. The process according to claim 8 or 9, wherein the at least one surfactant is added in an amount of from 2 to 10 g/L.

11. The process according to anyone of claims from 2 to 10, wherein the solubilizing step (b1) comprises adding an aqueous solution of a strong acid to the non-PHA cell mass.

12. The process according to claim 11, wherein the aqueous solution of a strong acid is added to the non-PHA cell mass in an amount so as to achieve a concentration of hydrogen ions (H⁺) from 0.01 to 0.5 mole/L.

13. The process according to anyone of claims from 2 to 12, wherein the solubilizing step (b1) is carried out at a temperature from 80° to 130°C, for a time from 0.5 to 5 hours.

14. The process according to anyone of claims 2 to 13, wherein the adjusting step (b2) comprises adding an aqueous solution of at least one strong base to the first suspension.

## Patentansprüche

1. Verfahren zur Herstellung von biologisch abbaubaren Polymerwerkstoffen einschließlich Polyhydroxyalkanoate (PHA) aus einer organischen Kohlenstoffquelle, umfassend:
(a) Kultivierung von PHA-erzeugenden Mikrobenzellen in einer Mediumlösung, die eine organische Kohlenstoffquelle zur Bildung von PHA enthält, die in den Zellen als Einschlusskörper angereichert sind;
(b) Entnahme der Zellen aus dem verbrauchten Medium und Solubilisierung der nicht-PHA-Zellmasse, um einen PHA-Feststoff und eine Zelltrümmerlösung zu gewinnen;
(c) Trennung des PHA-Feststoffes von der Zelltrümmerlösung;
(d) Einleitung der Zelltrümmerlösung in den Kultivierungsschritt (a).

2. Verfahren nach Anspruch 1, wobei der Solubilisierungsschritt (b) ausgeführt wird durch:
(b1) Solubilisierung der nicht-PHA-Zellmasse in einer sauren Lösung, um eine erste Suspension eines PHA-Feststoffes in einer sauren Zelltrümmerlösung zu gewinnen;
(b2) Einstellung des pH-Werts der ersten Suspension auf einen Wert zwischen 7 und 11, um eine zweite Suspension des PHA-Feststoffes in einer basischen Zelltrümmerlösung zu gewinnen.

3. Verfahren nach Anspruch 2, wobei ein Trennungsschritt (c1) bei der ersten Suspension ausgeführt und die dabei erhaltene saure Zelltrümmerlösung gemäß Schritt (d) zugeführt wird.

4. Verfahren nach Anspruch 3, wobei ein Trennungsschritt (c2) bei der zweiten Suspension ausgeführt und die dabei erhaltene basische Zelltrümmerlösung gemäß Schritt (d) zugeführt wird.

5. Verfahren nach Anspruch 2, wobei ein Trennungsschritt (c3) bei der zweiten Suspension ausgeführt und die dabei erhaltene Säure-Basen-Zelltrümmerlösung gemäß Schritt (d) zugeführt wird.

6. Verfahren nach einem beliebigen der vorstehenden Ansprüche, wobei die dem Kultivierungsschritt (a) zugeführte Gesamt-Zelltrümmermenge zwischen 5 und 50 Gewichtsprozent bezogen auf die Menge des glukoseäquivalenten Kohlenstoffsubstrats beträgt.

7. Verfahren nach Anspruch 3, wobei die dem Schritt (b) zugeführte Menge an saurer Zelltrümmerlösung (ACDS) zwischen 5 und 20 Gewichtsprozent bezogen auf das Gewicht des glukoseäquivalenten Kohlenstoffsubstrats beträgt.

8. Verfahren nach Anspruch 2, wobei der Einstellungsschritt (b2) die Anreicherung der ersten Suspension mit mindestens einem Oberflächenaktivstoff umfasst.

9. Verfahren nach Anspruch 8, wobei der mindestens ein Oberflächenaktivstoff ein ionischer Oberflächenaktivstoff ist.

10. Verfahren nach Anspruch 8 oder 9, wobei der mindestens ein Oberflächenaktivstoff in einer Menge zwischen 2 und 10 g/l angereichert wird.

11. Verfahren nach einem beliebigen Anspruch 2 bis 10, wobei der Solubilisierungsschritt (b1) die Anreicherung der nicht-PHA-Zellmasse mit einer wässrigen Lösung einer starken Säure umfasst.

12. Verfahren nach Anspruch 11, wobei die nicht-PHA-Zellmasse mit einer wässrigen Lösung einer starken Säure in einer derartigen Menge angereichert wird, dass eine Wasserstoffionenkonzentration (H⁺) zwischen 0,01 und 0,5 mol/l erreicht wird.

13. Verfahren nach einem beliebigen Anspruch 2 bis 12, wobei der Solubilisierungsschritt (b1) bei einer Temperatur zwischen 80° und 130°C über einen Zeitraum zwischen 0,5 und 5 Stunden ausgeführt wird.

14. Verfahren nach einem beliebigen Anspruch 2 bis 13, wobei der Einstellungsschritt (b2) die Anreicherung der ersten Suspension mit einer wässrigen Lösung von mindestens einer starken Base umfasst.

## Revendications

1. Procédé pour produire des matériaux polymériques biodégradables comprenant des polyhydroxyalcanoates (PHA) à partir d'une source de carbone organique, qui comprend :
(a) la culture de cellules microbiennes produisant des PHA dans une solution de milieu contenant une source de carbone organique pour former des PHA qui sont accumulés dans les cellules en tant que corps d'inclusion ;
(b) la récolte des cellules du milieu épuisé et la solubilisation de la masse cellulaire non PHA pour obtenir un solide PHA et une solution de débris cellulaires ;
(c) la séparation du solide PHA de la solution de débris cellulaires ;
(d) l'alimentation de la solution de débris cellulaires vers l'étape de culture (a).

2. Procédé selon la revendication 1, dans lequel l'étape de solubilisation (b) est exécutée par :
(b1) la solubilisation de la masse cellulaire non PHA dans une solution acide pour obtenir une première suspension de solide PHA dans une solution acide de débris cellulaires ;
(b2) l'ajustement du pH de la première suspension à une valeur de 7 à 11 pour obtenir une deuxième suspension du solide PHA dans une solution basique de débris cellulaires.

3. Procédé selon la revendication 2, dans lequel une étape de séparation (c1) est exécutée sur la première suspension et la solution acide de débris cellulaires résultante est alimentée conformément à l'étape (d).

4. Procédé selon la revendication 3, dans lequel une étape de séparation (c2) est exécutée sur la deuxième suspension et la solution basique de débris cellulaires résultante est alimentée conformément à l'étape (d).

5. Procédé selon la revendication 2, dans lequel une étape de séparation (c3) est exécutée sur la deuxième suspension et la solution acide-base de débris cellulaires résultante est alimentée conformément à l'étape (d).

6. Procédé selon l'une quelconque des revendications précédentes, dans lequel la quantité totale de débris cellulaires alimentés vers l'étape de culture (a) est de 5 à 50% en poids par rapport à la quantité du substrat de carbone équivalent-glucose.

7. Procédé selon la revendication 3, dans lequel la quantité de la solution acide de débris cellulaires (ACDS) alimentés vers l'étape (b) est de 5 à 20% en poids par rapport au poids du substrat de carbone équivalent-glucose.

8. Procédé selon la revendication 2, dans lequel l'étape d'ajustement (b2) comprend l'ajout d'au moins un agent tensioactif à la première suspension.

9. Procédé selon la revendication 8, dans lequel l'au moins un agent tensioactif est un agent tensioactif ionique.

10. Procédé selon la revendication 8 ou 9, dans lequel l'au moins un agent tensioactif est ajouté dans une quantité de 2 à 10 g/l.

11. Procédé selon l'une quelconque des revendications 2 à 10, dans lequel l'étape de solubilisation (b1) comprend l'ajout d'une solution aqueuse d'un acide fort à la masse cellulaire non PHA.

12. Procédé selon la revendication 11, dans lequel la solution aqueuse d'un acide fort est ajoutée à la masse cellulaire non PHA dans une quantité telle qu'elle permette d'atteindre une concentration d'ions d'hydrogène (H⁺) de 0,01 à 0,5 mole/1.

13. Procédé selon l'une quelconque des revendications 2 à 12, dans lequel l'étape de solubilisation (b1) est exécutée à une température de 80° à 130°C, pendant un temps de 0,5 à 5 heures.

14. Procédé selon l'une quelconque des revendications 2 à 13, dans lequel l'étape d'ajustement (b2) comprend l'ajout d'une solution aqueuse d'au moins une base forte à la première suspension.
